# EUROPEAN PATENT APPLICATION

(11) **EP 4 403 083 A1**
(43) Date of publication of application: **24.07.2024**
(21) Application number: 22870242.9
(22) Date of filing: 14.09.2022
(51) Int. Cl.: A47L 9/28, A47L 7/00, A47L 9/16, A47L 5/24, A61L 2/08, A47L 9/24, A47L 9/20

(54) **CLEANER STATION**

(30) Priority: 14.09.2021 KR 20210122622; 01.04.2022 KR 20220040917
(71) Applicant: LG Electronics Inc., Yeongdeungpo-gu Seoul 07336 (KR)
(72) Inventor: KIM, Hanshin, Seoul 08592 (KR); HYUN, Kietak, Seoul 08592 (KR); HER, Jonguk, Seoul 08592 (KR); CHO, Kyeongho, Seoul 08592 (KR); SONG, Hyunsup, Seoul 08592 (KR)
(74) Representative: Vossius & Partner Patentanwälte Rechtsanwälte mbB
(86) International application number: PCT/KR2022/013667
(87) International publication number: WO 2023/043158

(57) **Abstract**

Disclosed is a cleaner station including: a suction flow path having an inlet end communicating with a dust bin of a cleaner; and a dust collecting part connected to an outlet end of the suction flow path and collecting dust inside the dust bin of the cleaner. The dust collecting part includes: a dust collecting body having a dust inlet connected to the outlet end of the suction flow path; an inlet tube extending inward from a circumferential surface of the dust inlet and having an end forming a curvature circle with a specific curvature when viewed from one side; and an inlet tube cover disposed at the end of the inlet tube and opening and closing the inlet tube. Accordingly, by closing the inlet tube when the dust collecting part is separated from the cleaner station, the cleaner station may prevent dust from scattering, winged insects from escaping or odors from spreading.

## Description

### [Technical Field]

The present disclosure relates to a cleaner station, and more particularly, to a cleaner station that collects dust inside a cleaner and has a bagless type dust collecting part.

### [Background Art]

In general, a cleaner refers to an electrical appliance that draws in small garbage or dust by sucking air using electricity and fills a dust bin provided in a product with the garbage or dust. Such a cleaner is generally called a vacuum cleaner.

The cleaners may be classified into a manual cleaner which is moved directly by a user to perform a cleaning operation, and an automatic cleaner which performs a cleaning operation while autonomously traveling. Depending on the shape of the cleaner, the manual cleaners may be classified into a canister cleaner, an upright cleaner, a handy cleaner, a stick cleaner, and the like.

The canister cleaners were widely used in the past as household cleaners. However, recently, there is an increasing tendency to use the handy cleaner and the stick cleaner in which a dust bin and a cleaner main body are integrally provided to improve convenience of use.

In the case of the canister cleaner, a main body and a suction port are connected by a rubber hose or pipe, and in some instances, the canister cleaner may be used in a state in which a brush is fitted into the suction port.

The handy cleaner has maximized portability and is light in weight. However, because the handy cleaner has a short length, there may be a limitation to a cleaning region. Therefore, the handy cleaner is used to clean a local place such as a desk, a sofa, or an interior of a vehicle.

A user may use the stick cleaner while standing and thus may perform a cleaning operation without bending his/her waist. Therefore, the stick cleaner is advantageous for the user to clean a wide region while moving in the region. The handy cleaner may be used to clean a narrow space, whereas the stick cleaner may be used to clean a wide space and also used to a high place that the user's hand cannot reach. Recently, modularized stick cleaners are provided, such that types of cleaners are actively changed and used to clean various places.

Recently, cleaners tend to be miniaturized in order to maximize portability. Accordingly, recently produced cleaners have a small capacity of the dust bin, which is a hassle for a user to frequently empty the dust bin.

In order to solve the above-mentioned inconvenience, a cleaner station has been developed. The cleaner station is a device that mounts the cleaned cleaner and sucks the dust collected by the cleaner. In general, the cleaner station has a dust collecting part that is much larger than a size of the dust bin of the cleaner. That is, the dust collected by the cleaner is continuously collected in the dust collecting part of the cleaner station, and the user empties the dust collecting part of the cleaner station. According to this, there is an advantage in that the number of times the user emptying the dust is drastically reduced compared to the case of emptying the dust bin of the cleaner every time.

On the other hand, the dust collecting part is largely classified into a bag type and a bagless type. In the bag type, a separate bag is provided in the dust collecting part to collect dust from the bag, and only the bag is separated from the cleaner station and discarded. In contrast, the bagless type does not include a separate bag in the dust collecting part, collects dust in the dust collecting part itself, and separates the dust collecting part itself from the cleaner station to empty the dust. At this time, the bag is a one-time consumable, and since it is generally formed of a plastic material, there is an environmental problem that causes environmental pollution and a cost problem of having to purchase the bag every time. Therefore, in order to solve the above-mentioned disadvantages, a bagless type dust collecting part is often installed in the cleaner station.

In general, the dust collection interval of the bagless type dust collecting part is longer than that of the bag type dust collecting part. Accordingly, the dust collected inside the bagless type dust collecting part remains inside the dust collecting part for a considerable period of time. Accordingly, winged insects may appear in the collected dust, and when food or water remains, a problem of odor may occur.

The document of related art is an invention relating to a discharge station. According to the document of related art, when the robot cleaner is coupled to the discharge station, dust is sucked from the dustbin of the robot cleaner and collected in a bag. A latex membrane is disposed at a connection between a bag inlet and a suction pipe. When the bag is installed in the discharge station and an upper cover is closed, the suction pipe is inserted into the bag, and the suction pipe passes through the latex membrane, and the latex membrane is deformed. Accordingly, airtightness is maintained between the suction pipe and the bag.

According to the document of related art, considering the fact that the latex membrane is always installed at an inlet of the bag and the suction pipe is inserted through the latex membrane, it can be seen that the latex membrane has a hollow for the suction pipe to pass through. Accordingly, when the bag is separated from the discharge station, problems such as fine dust scattering from the hollow of the latex membrane, winged insects escaping, and odor spreading may occur.

In particular, since the bagless type has a larger capacity for storing dust than the bag type, the time for winged insects to breed or decay is long, and the problem is further exacerbated.

### [DISCLOSURE]

### [Technical Problem]

The problem to be solved by the present invention is to provide a cleaner station that prevents the dust collected inside the dust collecting part scattering to the outside, escape of winged insects and the like or the spread of odors, when the dust collecting part is separated from the cleaner station having a bagless type dust collecting part.

Another problem to be solved by the present invention is to provide a cleaner station that prevents the inlet tube cover for closing the inlet tube from sagging due to its own weight, dust scattering through the gap, escaping insects, or the spread of odor.

Another object to be solved by the present invention is to provide a cleaner station in which a gap is not generated by firmly bringing the inlet tube cover into closer contact with the inlet tube.

Still another object to be solved by the present invention is to provide a cleaner station that effectively kills microorganisms and winged insects present in the dust collecting part.

The problems of the present invention are not limited to the problems mentioned above, and other problems not mentioned will be clearly understood by those skilled in the art from the following description.

### [Technical Solution]

One embodiment is a cleaner station including: a suction flow path having an inlet end communicating with a dust bin of a cleaner; and a dust collecting part connected to an outlet end of the suction flow path and collecting dust inside the dust bin of the cleaner. The dust collecting part includes: a dust collecting body having a dust inlet connected to the outlet end of the suction flow path; an inlet tube extending inward from a circumferential surface of the dust inlet and having an end forming a curvature circle with a specific curvature when viewed from one side; and an inlet tube cover disposed at the end of the inlet tube and opening and closing the inlet tube.

The inlet tube may include: a first curvature circle; and a second curvature circle intersecting the first curvature circle.

The dust collecting part may include: a board disposed on an upper surface of the dust collecting body and having the dust inlet formed on one side thereof, and the inlet tube may extend downward from the board.

The dust collecting part may include: an inlet tube cover fixing member fixing one side of the inlet tube cover to the board and bringing the inlet tube cover into closer contact with an end of the inlet tube.

According to the first embodiment of the present disclosure, the dust collecting part may include: a rib protruding from the inlet tube cover fixing member and having an end thereof supporting the inlet tube cover. At this time, the rib may press and deform the inlet tube cover.

According to the second embodiment of the present disclosure, the inlet tube cover may include: a connecting portion coupled to the inlet tube; a closing portion for opening and closing an end of the inlet tube; and a bent portion disposed between the connecting portion and the closing portion. The inlet tube cover fixing member may include: an inlet tube cover insertion groove into which at least a portion of the inlet tube cover is inserted.

The dust collecting part may include: a side wall disposed at one lateral side of the dust inlet and extending into an inner space thereof.

The dust collecting part may include: a suction flow path sealer extending radially inward from the dust inlet and having an inner end in close contact with the suction flow path.

The cleaner station may further include: a sterilizing light emitting member disposed on an outside of the dust collecting body, and the dust collecting part may include: a sterilizing light irradiating part transmitting sterilizing light irradiated from the sterilizing light emitting member into the dust collecting body.

At this time, the gasket may be coupled to the inlet tube. Alternatively, the gasket may be coupled to the inlet tube cover and move together with the inlet tube cover.

Other details of the embodiments are included in the detailed description and the accompanying drawings.

### [Advantageous Effect]

According to the cleaner station of the present disclosure, there are one or more effects as below.

First, the cleaner station includes an inlet tube cover disposed at an end of the inlet tube for opening and closing the inlet tube, and as the inlet tube is closed by the cover when the dust collecting part is separated from the cleaner station, the cleaner station has the effect of preventing dust scattering to the outside, escape of winged insects and the like, and the spread of odors.

Second, the end of the inlet tube forms a curvature circle having a specific curvature, so there is no sagging due to its own weight, and there is an effect of tightly sealing the inlet tube.

Third, the dust collecting part includes a rib that deforms by pressing while supporting the inlet tube cover, the inlet tube cover is more firmly brought into closer contact with the end of the inlet tube while being deformed and is closely adhered thereto while forming a curvature.

Fourth, the inlet tube cover has an open front and side walls disposed on both sides of the inlet tube, and the air introduced through the inlet tube cannot flow backward by the inlet tube cover and cannot flow laterally by the side wall. Therefore, the air should flow forward, and since a sterilizing light irradiating part is disposed in front of the inlet tube to irradiate the sterilizing light, there is also an effect of effectively sterilizing and killing microorganisms and winged insects.

Effects of the present disclosure are not limited to the effects mentioned above, and other effects not mentioned will be clearly understood by those skilled in the art from the description of the claims.

### [Description of Drawings]

FIG. 1 is a perspective view of a cleaner station according to the present disclosure.
FIG. 2 is a view illustrating a suction flow path and a dust collecting part disposed inside the cleaner station in FIG. 1.
FIG. 3 is a perspective view viewed from the bottom after removing the dust collecting body from the cleaner station according to the first embodiment.
FIG. 4 is a front view of the cleaner station according to the first and second embodiments.
FIG. 5 is a right side view of the cleaner station according to the first and second embodiments.
FIG. 6 is an exploded view of the cleaner station according to the first embodiment.
FIG. 7 is a view of the bottom surface of the board of the cleaner station according to the first embodiment.
FIG. 8 is a side cross-sectional view of part B of FIG. 3 in the cleaner station according to the first embodiment.
FIG. 9 is a front cross-sectional view of part C of FIG. 4 in the cleaner station according to the first embodiment.
FIG. 10 is a state diagram before/after deformation of the inlet tube cover according to the first embodiment.
FIG. 11 is a diagram schematically showing the flow of air inside the dust collecting part according to the first embodiment.
FIG. 12 is a perspective view of the cleaner station according to the second embodiment as viewed from the bottom after removing the dust collecting body.
FIG. 13 is an exploded view of the cleaner station according to the second embodiment.
FIG. 14 is a view looking at the bottom surface of the board of the cleaner station according to the second embodiment.
FIG. 15 is a side cross-sectional view of part B of FIG. 3 in the cleaner station according to the second embodiment.
FIG. 16 is a front cross-sectional view of part C of FIG. 4 in the cleaner station according to the second embodiment.
FIG. 17 and FIG. 18 are a view showing a process in which the inlet tube cover is installed in the cleaner station according to the second embodiment.
FIG. 19 is a view showing that an inlet tube cover according to another embodiment is installed in the cleaner station according to the second embodiment.
FIG. 20 is an exploded view of the dust collecting part including an adhesive member in the cleaner station according to the second embodiment.
FIG. 21 is a cross-sectional view of the dust collecting part according to FIG. 20.
FIG. 22 is an exploded view of the dust collecting part including the gasket in the cleaner station according to the second embodiment.
FIG. 23 is a cross-sectional view of the dust collecting part according to FIG. 22.
FIG. 24 is a cross-sectional view of the gasket in a deformed state of FIG. 23.
FIG. 25 is a perspective view of a gasket according to another embodiment of the present invention.
FIG. 26 is a cross-sectional view of the dust collecting part including the gasket of FIG. 23.
FIG. 27 is a perspective view of a gasket according to still another embodiment of the present invention.

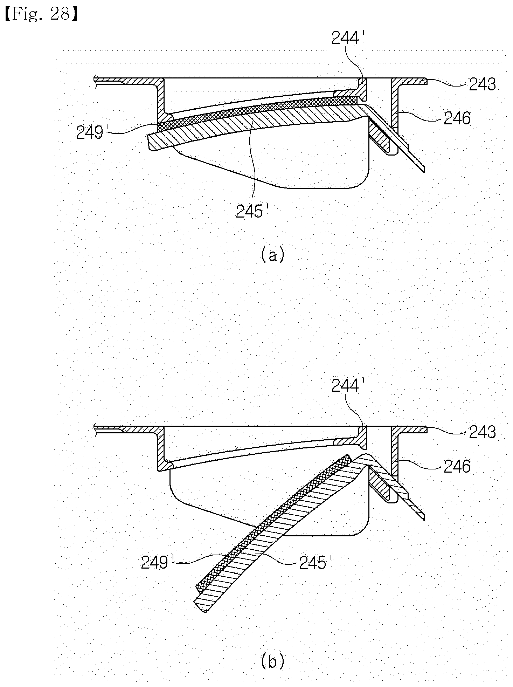

FIG. 28 is a cross-sectional view of the dust collecting part including the gasket of FIG. 27.

### [Detailed Description]

The merits and characteristics of the present disclosure and a method for achieving the merits and characteristics will become more apparent from the embodiments described in detail in conjunction with the accompanying drawings. However, the present disclosure is not limited to the disclosed embodiments, but may be implemented in various different ways. The embodiments are provided to only complete the disclosure of the present disclosure and to allow those skilled in the art to understand the category of the present disclosure. The present disclosure is defined by the category of the claims. The same reference numerals will be used to refer to the same or similar elements throughout the specification.

Hereinafter, the present disclosure is described through embodiments thereof in detail with reference to the accompanying drawings for explaining a cleaner station 100.

The cleaner system is divided into a cleaner 200 and a cleaner station 100. The cleaner is a movable component, moves and sucks dust present in a cleaning area and collects the dust in a dust bin provided therein. The cleaner sucks dust from the cleaned cleaner and collects it in the dust collecting parts 140 and 240 provided therein, which are fixed and non-movable components.

The cleaner 200 being coupled to the cleaner station 100 may include the cleaner being fixed to the cleaner station 100 and mechanically coupled, the cleaner being electrically coupled by contacting a terminal of the cleaner and a terminal of the cleaner station 100, and the cleaner being communicated so that air flows between the dust bin of the cleaner and the dust collecting parts 140 and 240 of the cleaner station 100.

The cleaner 200 may mean a cleaner manually operated by a user. For example, the cleaner 200 may mean a handy cleaner or a stick cleaner.

The cleaner 200 may include a main body 210. The main body 210 includes a main body housing 211, a suction part 212, a dust separation part 213, a suction motor 214, an air exhaust cover 215, a handle 216, an extension part 217, and an operator unit 218.

The main body housing 211 may form an exterior of the cleaner 200. The main body housing 211 may provide a space for accommodating the suction motor 214 and a filter (not illustrated) therein. The main body housing 211 may be configured in a shape similar to a cylinder.

The suction part 212 may protrude outward from the main body housing 211. For example, the suction part 212 may be formed in a cylindrical shape. The suction part 212 may communicate with an extension pipe 250. The suction part 212 may provide a flow path through which air containing dust may flow.

The dust separation part 213 may communicate with the suction unit 212. The dust separation part 213 may separate dust sucked into the inside through the suction part 212. The dust separation part 213 may communicate with the dust bin 220.

The dust separation part 213 may be a cyclone capable of separating dust by cyclone flow.

The suction motor 214 may generate a suction force for sucking air. The suction motor 214 may be accommodated in the main body housing 211. The suction motor 214 may generate suction force by rotation. For example, the suction motor 214 may be provided similarly to a cylindrical shape.

The air exhaust cover 215 may be disposed on the main body housing 211. A filter for filtering air may be accommodated in the air exhaust cover 215. For example, a HEPA filter may be accommodated in the air exhaust cover 215.

The handle 216 may be gripped by a user. The handle 216 may be disposed behind the suction motor 214. For example, the handle 216 may be formed similarly to a cylindrical shape. Alternatively, the handle 216 may be formed in a curved cylindrical shape. The handle 216 may be disposed at a predetermined angle with the main body housing 211, the suction motor 214, or the dust separation part 213.

The extension part 217 may extend from the handle 216 toward the main body housing 211. At least a portion of the extension part 217 may extend in a horizontal direction.

The operator unit 218 may be disposed on the handle 216. The operator unit 218 may be disposed on an inclined surface formed in an upper region of the handle 216. The user may input a command of operation or stop of the first cleaner 200 through the operator unit 218.

The cleaner 200 may include a dust bin 220. The dust bin 220 may communicate with the dust separation part 213. The dust bin 220 may store the dust separated by the dust separation part 213.

The cleaner station 100 is a component for charging the cleaner, sucking the dust collected in the dust bin of the cleaner, or safely storing the cleaner after cleaning, by being coupled to the cleaner 200.

The cleaner station 100 includes a housing 110. The housing 110 forms an outer shape of the cleaner station 100 and forms a space in which other components are accommodated. The housing 110 may be formed in a pillar shape including at least one outer wall surface. For example, the housing 110 may be formed in a shape similar to a rectangular pole.

A coupling member 120 is disposed in the housing 110. The coupling member 120 is a component that supports the cleaner when the cleaner is coupled to the cleaner station 100.

In the suction flow path 130, an inlet end communicates with the dust bin (not illustrated) of the cleaner, and an outlet end communicates with an inlet tube 144 of the dust collecting parts 140 and 240. The suction flow path 130 is a pipe or tube having a hollow so that air containing dust flows therein.

A dust collecting motor 190 is disposed in the housing 110. The dust collecting motor forms a negative pressure to form an air flow in the suction flow path 130 or the discharge flow path (not illustrated). The dust collecting motor communicates with the suction flow path 130 and the discharge flow path (not illustrated).

A filter (not illustrated) is disposed in the housing 110. For example, the filter may be disposed between the dust collecting parts 140 and 240 and the dust collecting motor.

The dust collecting parts 140 and 240 are components that collect dust inside the dust bin of the cleaner. The dust collecting parts 140 and 240 are accommodated in the housing 110 of the cleaner station 100.

The dust collecting parts 140 and 240 according to the present invention are of a bagless type. The dust collecting parts 140 and 240 are largely classified into a bag type and a bagless type. In the bag type, a separate bag is provided in the dust collecting parts 140 and 240 to collect dust from the bag, and only the bag is separated from the cleaner station 100 and discarded. In contrast, the bagless type does not include a separate bag in the dust collecting parts 140 and 240, and the dust collecting parts 140 and 240 thereof collect dust, and the bagless type empties dust by separating the dust collecting parts 140 and 240 from the cleaner station 100.

The dust collecting parts 140 and 240 according to the present invention are detachably disposed in the cleaner station 100. The dust collecting parts 140 and 240 may be separated from the cleaner station 100 so that the collected dust is emptied and the inside thereof is cleaned. When the dust collecting parts 140 and 240 are separated from the cleaner station 100, the connection between the suction flow path 130 and dust inlets 1412, 1422 and 1432 is released. Therefore, through the dust inlet 1412, 1422 and 1432, problems such as winged insects escaping to the outside, dust scattering, or spread of the odor may occur. Accordingly, for the purpose of solving the abovementioned problems, the dust collecting parts 140 and 240 according to the present invention are provided with the inlet tube covers 145 and 245, so that problems such as the winged insects escaping to the outside, the dust scattering through the dust inlets 1412, 1422, and 1432, or the spread of odors have been solved.

The dust collecting parts 140 and 240 according to the present invention may include the dust collecting part 140 according to the first embodiment and the dust collecting part 240 according to the second embodiment. Hereinafter, the dust collecting part 140 according to the first embodiment will be described first, and the dust collecting part 240 according to the second embodiment will be described focusing on differences from the dust collecting part 240 according to the first embodiment.

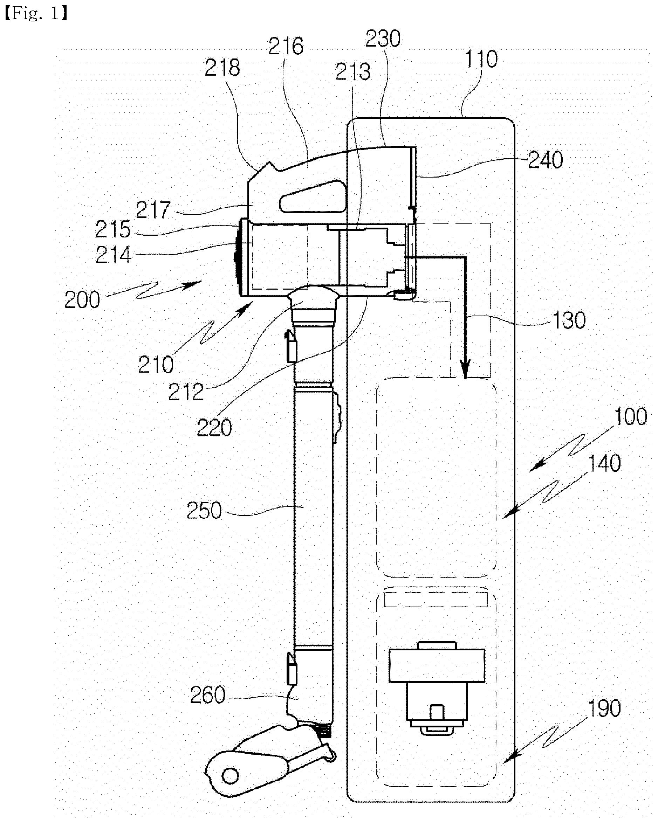

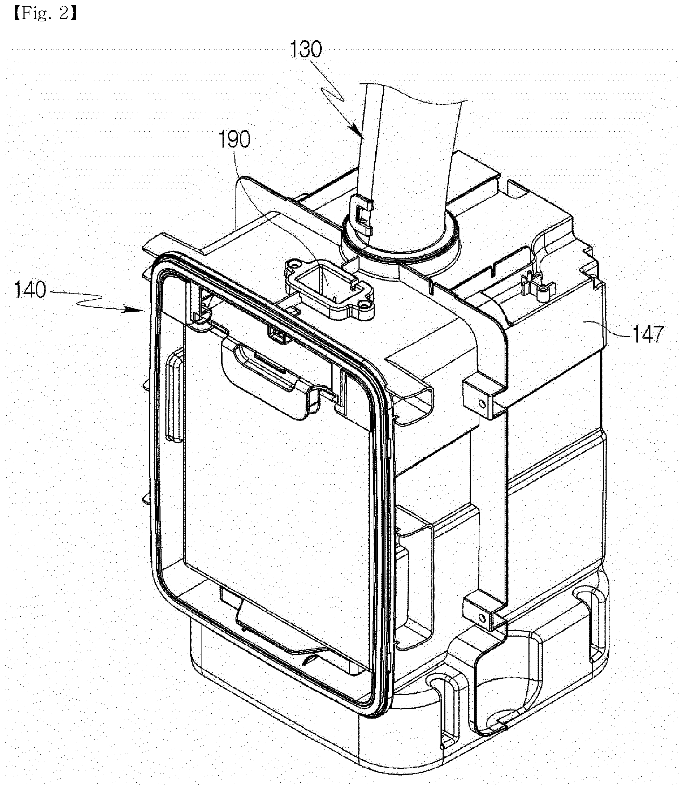

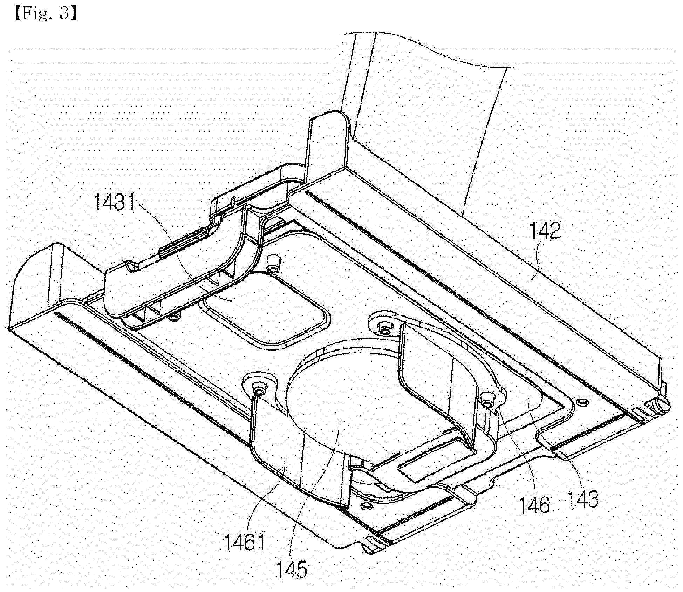

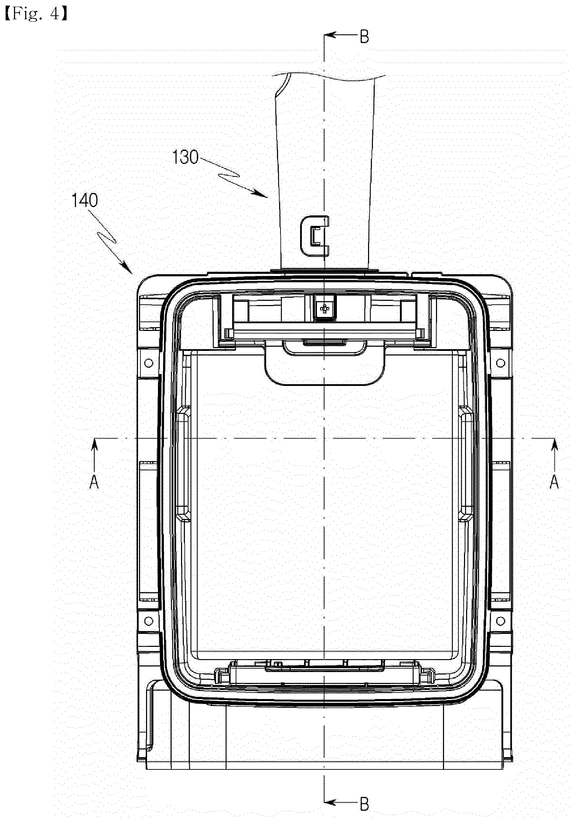

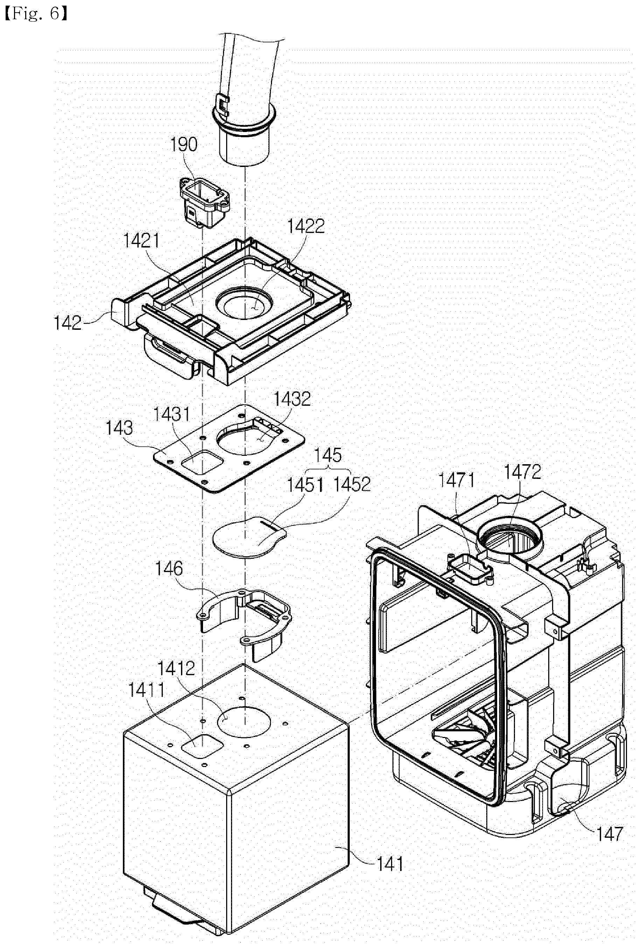

With reference to FIGS. 1 to 10, the dust collecting part 140 according to the first embodiment will be described. The dust collecting part 140 according to the first embodiment includes a dust collecting body 141, an outer board 142, an inner board 143, and an inlet tube 144. The dust collecting body 141, the outer board 142, and the inner board 143 are disposed inside a dust collecting part case 147.

The dust collecting part case 147 forms an outer shape of the dust collecting part 140, and forms a space in which each component of the dust collecting part 140 is accommodated.

The front side of the dust collecting part case 147 is opened, and each component of the dust collecting part 140 is inserted into the front side.

The dust collecting part case includes a light emitting member installation part 1471. The light emitting member installation part 1471 forms a space in which a sterilization light emitting member is installed. The light emitting member installation part 1471 may be formed on an upper wall of the dust collecting part case 147.

The dust collecting part case 147 includes a dust inlet 1472. The dust inlet 1472 is a hole penetrating the dust collecting part case 147 up and down. The dust inlet 1472 of the dust collecting part case 147 communicates with the dust inlet 1422 of the outer board 142, the dust inlet 1432 of the inner board 143, and the dust inlet 1412 of the dust collecting body 141. The suction flow path 130 is inserted into the dust inlet 1472 of the dust collecting part case 147.

The dust collecting body 141 forms an outer shape of the dust collecting part 140 and has an inner space in which dust can be collected.

The dust collecting body 141 is detachably mounted inside the housing 110 of the cleaner station 100. Specifically, the dust collecting body 141 is attached and detached from a front of the housing 110 of the cleaner station 100.

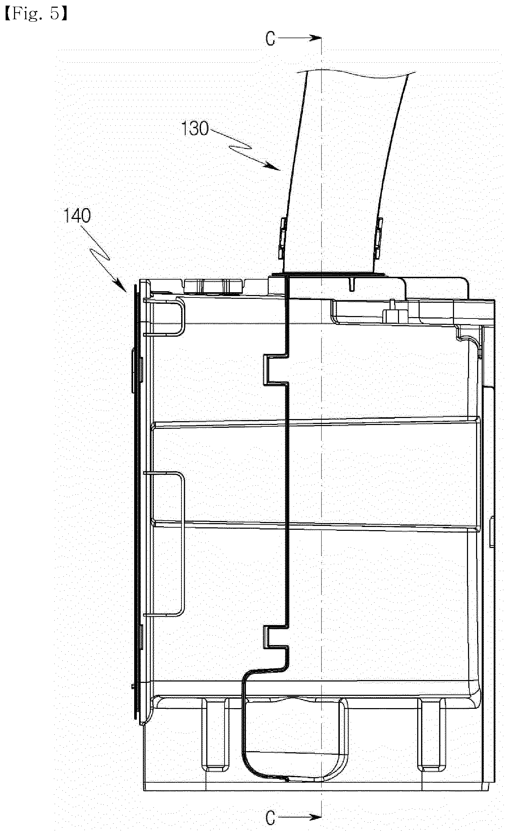

Referring to FIG. 5, the dust collecting body 141 is formed in a shape similar to a hexahedron. However, the present invention is not limited thereto, and may be formed in other polyhedral or cylindrical shapes.

The dust inlet 1412 is formed in the dust collecting body 141. The dust inlet 1412 of the dust collecting body 141 is formed on an upper surface thereof.

Alternatively, the upper surface of the dust collecting body 141 may be opened, and a board on which the dust inlet 1412 is formed may be disposed on the upper surface of the dust collecting body 141.

The dust inlet 1412 of the dust collecting body 141 is disposed to overlap with the dust inlet 1422 of the outer board 142 and the dust inlet 1432 of the inner board 143. Accordingly, the dust inlet 1412 of the dust collecting body 141 communicates with the dust inlet 1422 of the outer board 142 and the dust inlet 1432 of the inner board 143.

Hereinafter, the dust inlets 1412 1422 and 1432 may include the dust inlet 1412 of the dust collecting body 141, the dust inlet 1422 of the outer board 142, and the dust inlet 1432 of the inner board 143 unless they conflict with each other.

The dust collecting body 141 includes a sterilizing light irradiating part 1411. The sterilizing light irradiating part 1411 transmits the sterilizing light. The sterilizing light irradiating part 1411 may be formed to protrude or sink from the dust collecting body 141 in order to diffuse or converge the sterilizing light.

The sterilizing light irradiating part 1411 of the dust collecting body 141 is disposed to overlap vertically with the light emitting member installation part 1471 of the dust collecting part case 147, the sterilizing light irradiating part 1411 of the dust collecting part 141, and the sterilizing light irradiating part 1431 of the inner board 143.

The boards 142 and 143 are disposed on an upper surface of the dust collecting part 140. The board has dust inlets 1422 and 1432 formed on one side.

The board includes the outer board 142 and the inner board 143.

The outer board 142 is disposed on an outer surface of the upper wall of the dust collecting body 141. The outer board 142 may be vertically disposed on the suction flow path 130.

The outer board 142 includes the dust inlet 1422. The dust inlet 1422 is a hole penetrating the outer board 142 up and down. The dust inlet 1422 of the outer board 142 communicates with the dust inlet 1432 of the inner board 143 and the dust inlet 1412 of the dust collecting body 141. The suction flow path 130 may be inserted into the dust inlet 1422 of the outer board 142.

The outer board 142 includes the sterilizing light irradiating part 1421. The sterilizing light irradiating part 1421 transmits the sterilizing light. The sterilizing light irradiating part 1421 may be formed by protruding or being recessed from the outer board 142 in order to diffuse or converge the sterilizing light.

The sterilizing light irradiating part 1421 of the outer board 142 is disposed to overlap vertically with the light emitting member installation part 1471 of the dust collecting part case 147, the sterilizing light irradiating part 1411 of the dust collecting body 141 and the sterilizing light irradiating part 1431 of the inner board 143.

The board includes a suction flow path sealer 1423. The suction flow path sealer 1423 is a component that maintains airtightness between the suction flow path 130 and the dust collecting part 140. The suction flow sealer 1423 seals a gap between the dust inlets 1422 and 1432 and the suction flow path 130 to block foreign substances such as dust from scattering into the gap.

The suction flow path sealer 1423 may be disposed on the outer board 142. Alternatively, the suction flow path sealer 1423 may be disposed on the inner board 143.

The suction flow path sealer 1423 extends radially inward from the dust inlets 1422 and 1432, and an inner end thereof is in close contact with the suction flow path 130.

A diameter of an inner circumferential surface of the suction flow path sealer 1423 may be formed smaller than a diameter of an outer circumferential surface of the suction flow path 130. Accordingly, when the suction flow path 130 is inserted into the dust inlets 1422 and 1432, the suction flow path sealer 1423 may be deformed inwardly and more firmly brought into closer contact with the outer circumferential surface of the suction flow path 130.

The inner board 143 is disposed on an inner surface of an upper wall of the dust collecting body 141. The inner board 143 may be vertically disposed on the suction flow path 130.

The inner board 143 includes the dust inlet 1432. The dust inlet 1432 is a hole penetrating the inner board 143 up and down. The dust inlet 1432 of the inner board 143 communicates with the dust inlet 1422 of the outer board 142 and the dust inlet 1412 of the dust collecting body 141.

The inner board 143 includes the sterilizing light irradiating part 1431. The sterilizing light irradiating part 1431 transmits the sterilizing light. The sterilizing light irradiating part 1431 may be formed to protrude or be recessed from the inner board 143 in order to diffuse or converge the sterilizing light.

The sterilizing light irradiating part 1431 of the inner board 143 is disposed to overlap vertically with the light emitting member installation part 1471 of the dust collecting part case 147, the sterilizing light irradiating part 1411 of the dust collecting body 141 and the sterilizing light irradiating part 1421 of the outer board 142.

The inlet tube 144 is disposed on the inner board 143.

The inlet tube 144 is a component that communicates with the suction flow path 130 and guides the dust flowing through the suction flow path 130 into the dust collecting body 141.

The inlet tube 144 extends inward from circumferential surfaces of the dust inlets 1412, 1422 and 1432, and forms a curvature circle having a specific curvature at an end thereof when viewed from one side. Specifically, the inlet tube 144 extends downward from the dust inlet 1432 of the inner board 143.

The inlet tube 144 includes a first curvature circle C1 and a second curvature circle C2. The second curvature circle intersects the first curvature circle.

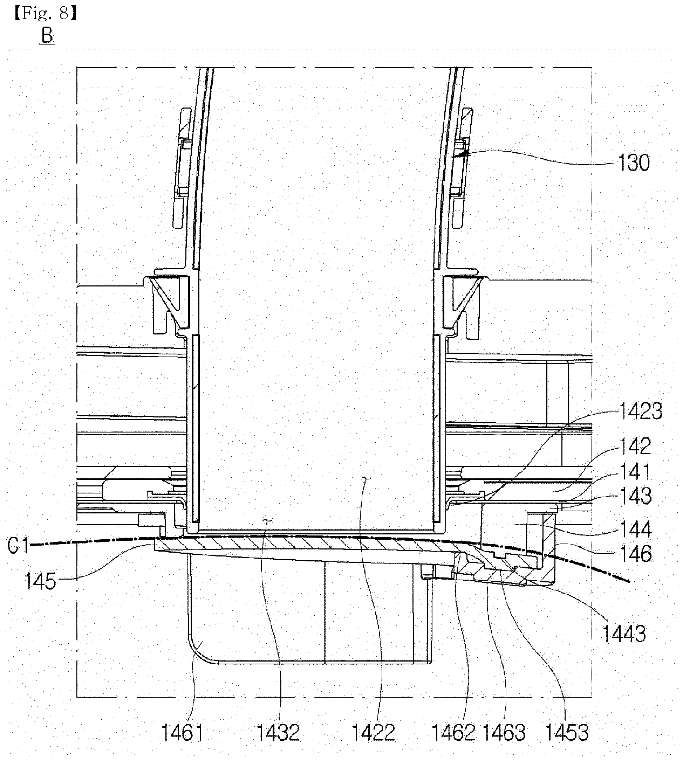

Referring to FIG. 8, when viewed from a right side, a lower end of the inlet tube 144 forms a curved surface. The curved surface follows a trajectory of the first curvature circle. The first curvature circle is an imaginary curve extending forward and backward. A first center of curvature is disposed at a lower portion with respect to the cross-sectional area of the inlet tube 144. When the inlet tube 144 cover is in close contact with the inlet tube 144, the inlet tube 144 is deformed into a convex shape upward so that a center is disposed higher than a front end and a rear end.

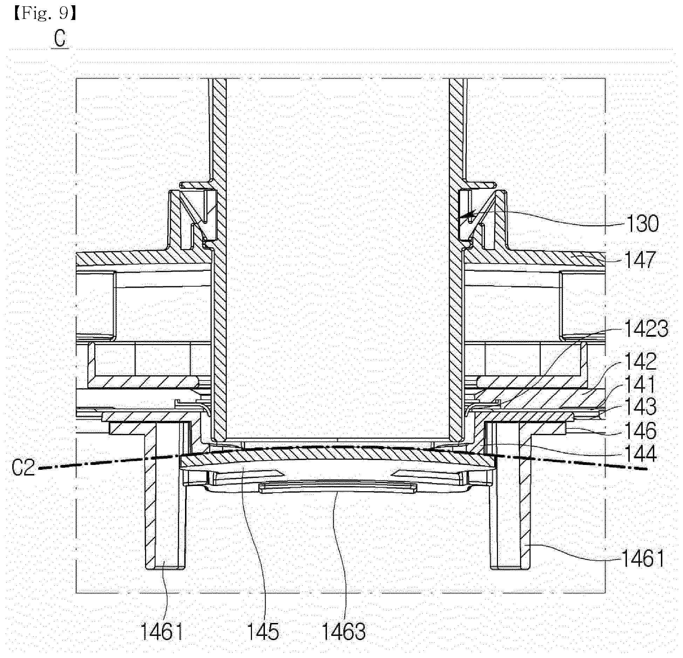

Referring to FIG. 9, when viewed from a front, a lower end of the inlet tube 144 forms a curved surface. The curved surface follows a trajectory of the second curvature circle. The second curvature circle is an imaginary curve extending to left and right sides. A second center of curvature is disposed at a lower portion with respect to the cross-sectional area of the inlet tube 144. When the inlet tube cover 145 is in close contact with the inlet tube 144, the inlet tube 144 is deformed into a convex shape upward so that the center is disposed higher than the left end and the right end.

The first curvature circle intersects the second curvature circle. For example, the first curvature circle may extend forward and backward, while the second curvature circle may extend leftward and rightward. Accordingly, when a front end of the inlet tube cover 145 is about to sag downward by its own weight, the front end of the inlet tube cover 145 does not sag downward due to the deformation according to the second curvature circle. Conversely, when a left or right end of the inlet tube cover 145 is about to sag downward due to its own weight, the front end of the inlet tube cover 145 does not sag downward due to deformation according to the first curvature circle.

A first step 1443 is formed in the inlet tube 144. The first step 1443 is formed to further protrude downward from a lower end of the inlet tube 144. The first step 1443 is inserted into a groove formed on an upper surface of the second step 1453 of the inlet tube cover 145.

The inlet tube cover 145 is a component for opening and closing the inlet tube 144.

The inlet tube cover 145 is disposed at an end of the inlet tube 144.

Referring to FIG. 8, when viewed from the right side, the inlet tube cover 145 is in close contact with the inlet tube 144 and is deformed to have a first curvature.

Referring to FIG. 9, when viewed from the front, the inlet tube cover 145 is in close contact with the inlet tube 144 and is deformed to have a second curvature.

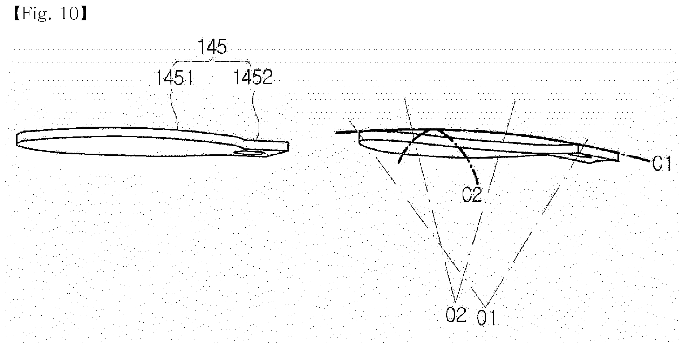

Referring to FIG. 10, the inlet tube cover 145 is manufactured in a flat plate shape, but is in close contact with an end of the inlet tube 144 and is deformed to have the first curvature and the second curvature. The first curvature and the second curvature are different from each other.

The inlet tube cover 145 according to the first embodiment may be divided into a connecting portion 1451 and a closing portion 1452. The connecting portion 1451 and the closing portion 1452 are integrally formed.

The closing portion 1452 is in close contact with an end of the inlet tube 144 to open and close the inlet tube 144. The closing portion 1452 is formed in a shape corresponding to a cross-sectional area of the inlet tube 144. For example, the closing portion 1452 may be formed as a circular plate.

The connecting portion 1451 is disposed on one side of the closing portion 1452, and fixes the closing portion 1452 to the inner board 143. Specifically, the connecting portion 1451 may be formed at a rear end of the closing portion 1452. The connecting portion 1451 may be fitted into and coupled to the inlet tube 144 and the inlet tube cover fixing member 146 and heat-sealed.

The inlet tube cover 145 may include a second step 1453. The second step 1453 is formed to further protrude downward from the connecting portion 1451 of the inlet tube cover 145. A lower end of the second step 1453 is inserted into a groove formed in an upper surface of a third step 1463 of the inlet tube cover fixing member 146. A groove into which the first step 1443 is inserted may be formed at an upper end of the second step 1453. The lower end of the second step 1453 is inserted and fixed into the groove formed on the upper portion of the third step 1463, and the groove formed at an upper end of the second step 1453 is fixed as the lower end of the first step 1443 is inserted thereinto.

The inlet tube cover 145 may further protrude outward in a radial direction of the inlet tube 144. Referring to FIG. 8, the inlet tube cover 145 further protrudes forward of the inlet tube 144. Referring to FIG. 9, the inlet tube cover 145 further protrudes to the left or right of the inlet tube 144. A diameter of the inlet tube cover 145 is larger than a diameter of an outer circumferential surface of the inlet tube 144, and the inlet tube cover 145 may cover all of the lower surface of the inlet tube 144.

The inlet tube cover 145 may be formed of a flexible rubber material, and the inlet tube 144 may be formed of a hard resin material. In this case, as the inlet tube cover 145 protrudes further outward in a radial direction of the inlet tube 144, a contact area with the inlet tube 144 increases as the inlet tube cover 145 is depressed inward, thereby further improving a sealing effect.

In addition, when the dust collecting motor 190 is operated, an effective area over which the negative pressure generated by the dust collecting motor 190 is applied to the inlet tube cover 145 increases, thereby the inlet tube cover 145 is more easily opened.

The inlet tube cover fixing member 146 is a component for coupling the inlet tube cover 145 to the dust collecting body 141 or the board. The inlet tube cover fixing member 146 fixes one side of the inlet tube cover 145 to the board, and brings the inlet tube cover 145 into closer contact with an end of the inlet tube 144.

The connecting portion 1451 of the inlet tube cover 145 is inserted into the inlet tube cover fixing member 146. A lower surface of the connecting portion 1451 is supported by the inlet tube cover 145, and an upper surface of the connecting portion 1451 is supported by the inlet tube 144.

The inlet tube cover 145 may include the third step 1463. The third step 1463 is formed to further protrude downward from the inlet tube cover fixing member 146. An upper end of the third step 1463 is formed with a groove so that the second step is inserted thereto.

The first step 1443 is inserted into a groove formed in an upper portion of the second step 1453, and a lower end of the second step 1453 is inserted into a groove formed in an upper portion of the third step 1463, so that the inlet tube cover 145 can be fixed in a correct position. Thereafter, the inlet tube cover 145, the inlet tube 144, and the inlet tube cover fixing member 146 are firmly coupled by heat sealing.

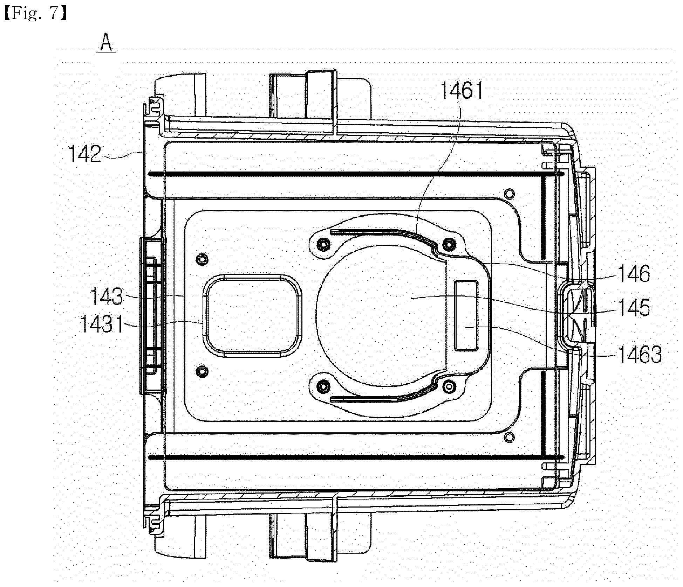

Referring to FIG. 7, the dust collecting part 140 includes a rib 1462. The rib 1462 supports the inlet tube cover 145, specifically, the rib 1462 deforms a shape of the inlet tube cover 145 to form a curvature.

The rib 1462 protrudes from the inlet tube cover fixing member 146, and an end thereof supports the inlet tube cover 145.

The rib 1462 is deformed by pressing the inlet tube cover 145. A lower surface of the inlet tube cover 145 is supported on an inner surface of the inlet tube cover fixing member 146. The rib 1462 is formed to further protrude upward from the inner surface of the inlet tube cover fixing member 146, and presses one side of the lower surface of the inlet tube cover 145 upward, and a portion of the inlet tube cover 145 in contact with the rib 1462 is deformed to become convex upward.

The rib 1462 is disposed radially outward of the suction flow path 130. The rib 1462 extends along a circumferential direction of the suction flow path 130.

The ribs 1462 are disposed radially inward of the inlet tube 144.

The rib 1462 is disposed radially inward than the third step 1463. The rib 1462, the inner surface of the inlet tube cover fixing member 146, and the third step 1463 may be formed to have a two-step stepped portion. Therefore, they are prevented as much as possible from being torn or damaged when the inlet tube cover 145 is deformed.

The dust collecting part 140 includes a side wall 1461. The side wall 1461 is a component for limiting and guiding a flow direction of the air introduced into the dust collecting body 141. The side wall 1461 is disposed on one side of the dust inlet 1432 and extends into an inner space.

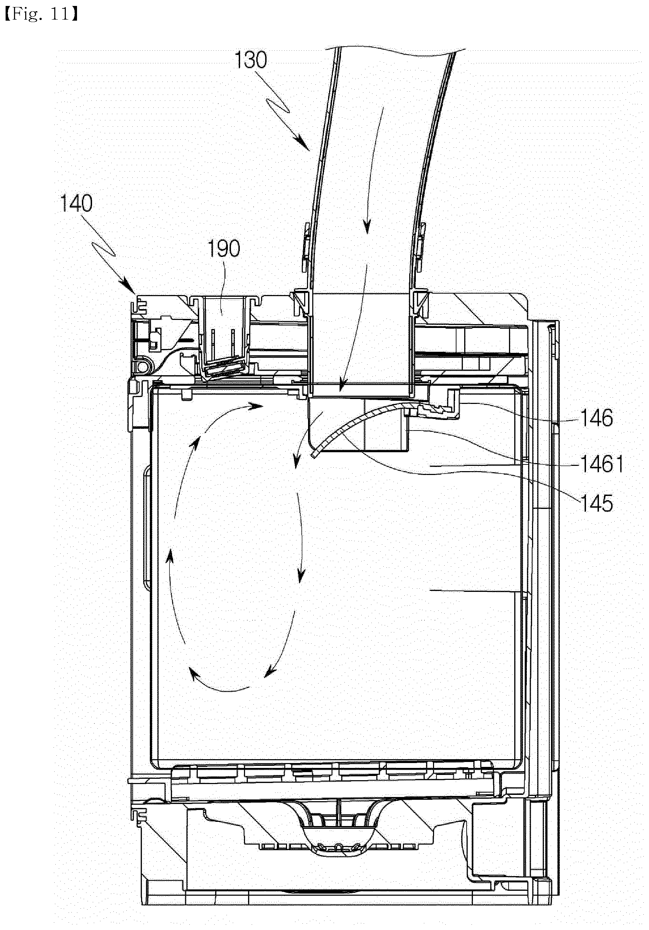

The side wall 1461 is symmetrically disposed on the left and right sides of the inlet tube 144. Accordingly, the side wall 1461 blocks the air introduced into the inlet tube 144 from flowing to the left or right. In addition, the inlet tube cover 145 blocks the air introduced into the inlet tube 144 from flowing backward since the connecting portion 1451 is disposed at a rear and a front end thereof is opened. Accordingly, as illustrated in FIG. 11, the air introduced into the inlet tube 144 flows forward and downward.

Referring to FIG. 11, the air introduced into the inlet tube 144 flows forward and downward. The sterilizing light emitting member 150 and sterilizing light irradiating part 1411/1421/1431 are disposed in front of the inlet tube 144, and sterilizing light is irradiated to the front of the inlet tube 144. Accordingly, microorganisms and winged insects included in the air introduced into the inlet tube 144 are sterilized or killed by the sterilizing light.

The cleaner station 100 includes a sterilizing light emitting member 150. The sterilizing light emitting member is disposed outside the dust collecting body 141. The sterilizing light emitting member irradiates sterilizing light into the dust collecting body 141.

The sterilizing light may be Ultra-Violet (UV) light. The sterilizing light is a known light, and since it is light of a wavelength that can be easily adopted by a person skilled in the art, a detailed description thereof will be omitted.

The dust collecting part 140 includes sterilizing light irradiating parts 1421 and 1431. The sterilizing light irradiating parts 1421 and 1431 transmit the sterilizing light irradiated from the sterilizing light emitting member into the dust collecting body 141.

The sterilizing light irradiating parts 1421 and 1431 may be formed on the dust collecting body 141, the outer board 142, and the inner board 143.

The sterilizing light irradiating parts 1421 and 1431 may be formed of a transparent or translucent material to transmit the sterilizing light.

The sterilizing light irradiating parts 1421 and 1431 may be depressed inward or protrude outward from the upper board to diffuse or converge the sterilizing light.

The sterilizing light irradiating parts 1421 and 1431 may be disposed in front of the inlet tube 144. The air introduced into the inlet tube 144 flows forward and downward, and the sterilizing light irradiated to the inside through the sterilizing light irradiating parts 1421 and 1431 may kill microorganisms and winged insects included in the introduced air.

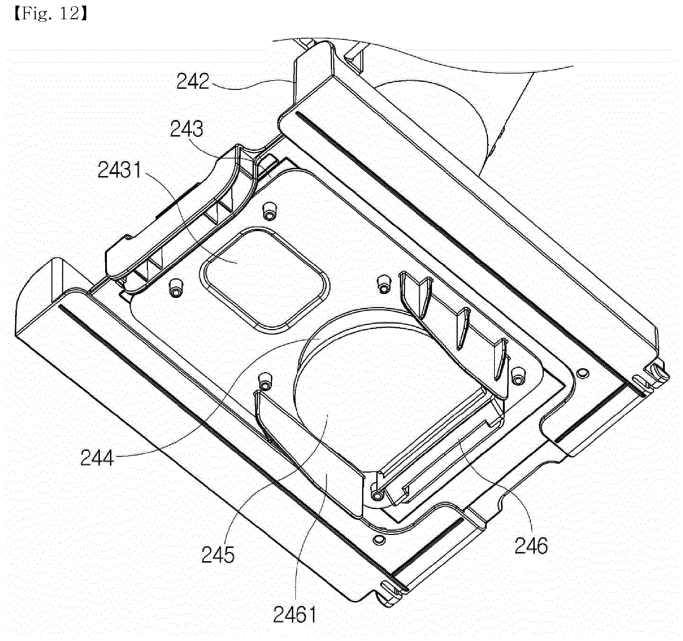

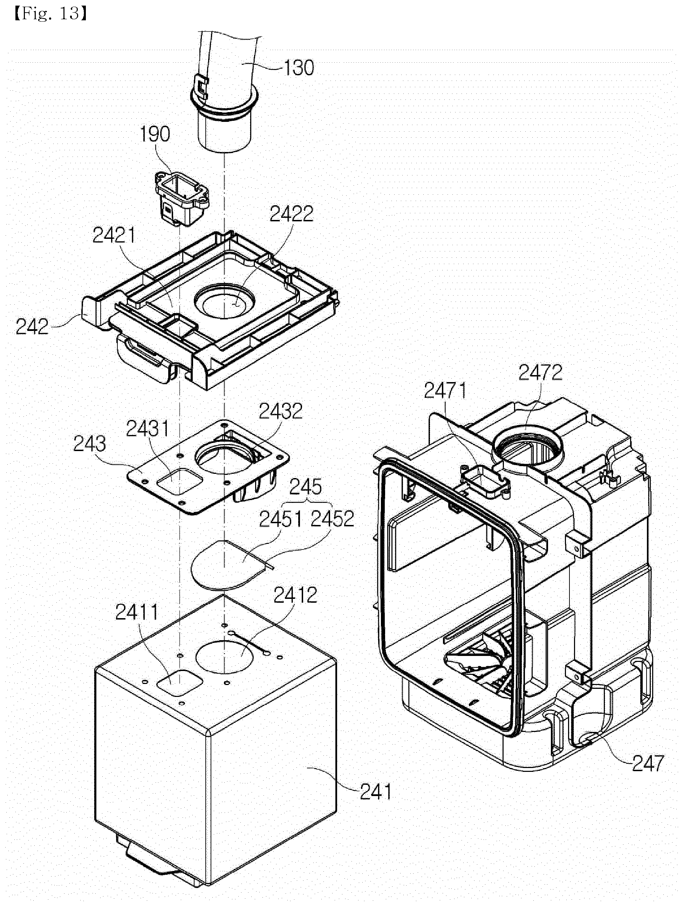

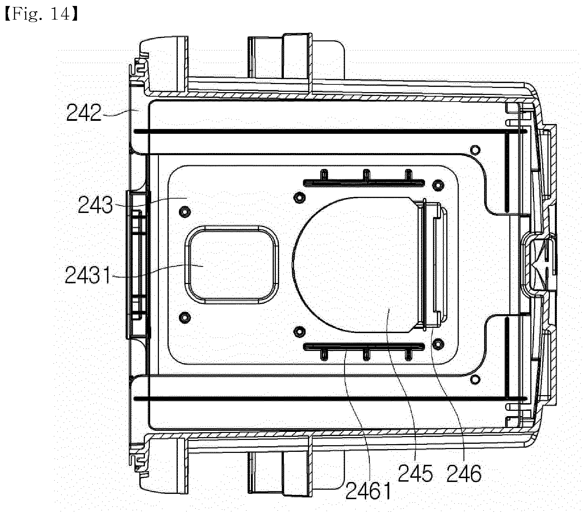

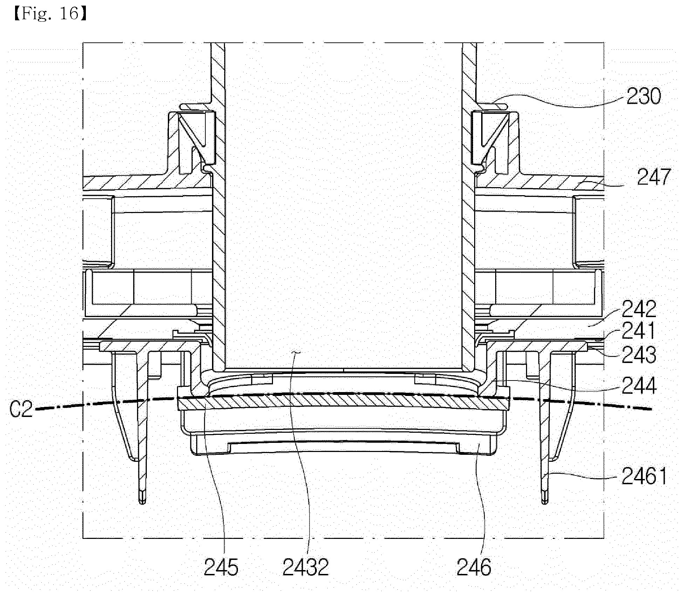

Hereinafter, a dust collecting part 240 according to the second embodiment will be described with reference to FIGS. 11 to 17. The description of the dust collecting part 240 according to the second embodiment may be used within a range that does not conflict with the dust collecting part 140 according to the first embodiment described above, and a description of the same features is omitted. Hereinafter, differences between the dust collecting part 240 according to the second embodiment and the dust collecting part 140 according to the first embodiment will be mainly described.

The inlet tube cover 245 according to the second embodiment may be divided into a connecting portion 2451, a closing portion 2452, and a bent portion 2453.

The connecting portion 2451 is coupled to the inlet tube 244. The connecting portion 2451 is disposed on one side of the closing portion 2452, and fixes the closing portion 2452 to the inlet tube cover fixing member 146. Specifically, the connecting portion 2451 is inserted into and coupled to the inlet tube cover fixing member 146, and heat-sealed.

The closing portion 2452 opens and closes an end of the inlet tube 244. The closing portion 2452 is formed in a shape corresponding to a cross-sectional area of the inlet tube 244 and is not different from that of the inlet tube cover 15 according to the first embodiment.

The bent portion 2453 is disposed between the connecting portion 2451 and the closing portion 2452. An inclination of the connecting portion 2451 and an inclination of the closing portion 2452 with respect to the bent portion may be different from each other. For example, with respect to the bent portion, the connecting portion 2451 has a rear downward inclined surface, and the closing portion 2452 has a forward downward inclined surface.

The inlet tube cover 245 has a restoring force by itself, since the connecting portion 2451 and the closing portion 2452 are deformed with respect to the bent portion.

The inlet tube cover fixing member 146 is manufactured integrally with the inner board 143. Alternatively, the inlet tube cover fixing member 146 may be manufactured separately from the inner board 143 and then combined.

The inlet tube cover fixing member 146 includes an inlet tube cover insertion groove 2462. At least a portion of the inlet tube cover 245 is inserted into the inlet tube cover insertion groove and fixed thereto.

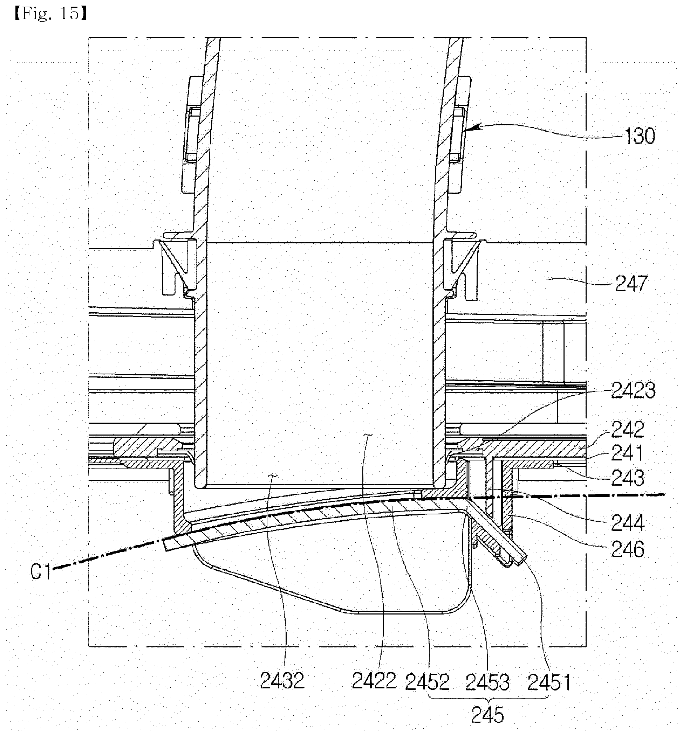

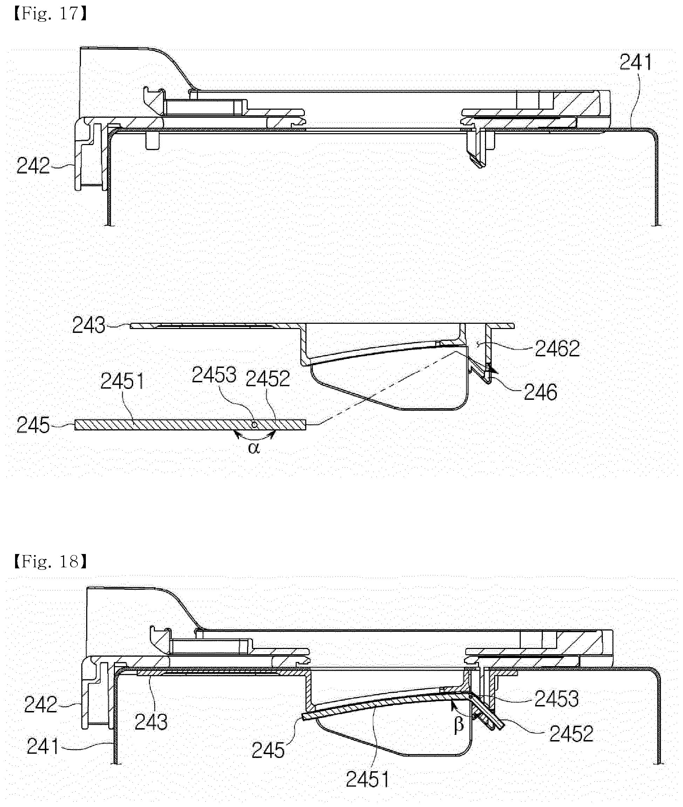

Referring to FIGS. 15 and 17, the inlet tube cover insertion groove has a rear downward inclined surface and may extend in the left and right directions. Accordingly, the inlet tube cover 245 is inserted downwardly from a front upper portion to the rear.

Referring to FIG. 17, the inlet tube cover 245 is formed in a flat plate shape, and may be mounted while being deformed. Referring to FIG. 17a, the inlet tube cover 245 in a state before mounting is formed to be flat. On the other hand, referring to FIG. 17b, the inlet tube cover 245 in a state after mounting is formed in a V-shape with respect to the bent portion 2453. Accordingly, the closing portion 2452 may be in close contact with a lower end of the inlet tube 244 while restoring force is applied upward.

Referring to FIG. 17, the inlet tube cover 245 is coupled to a rear end of the inlet tube 244, and a front end of the inlet tube 244 is disposed below the rear end of the inlet tube 244. As the inlet tube cover 245 is coupled to the rear end of the inlet tube 244, sagging occurs at a front end of the inlet tube cover 245, so that the front end of the inlet tube cover 245 falls first from the inlet tube 244, which is a problem. At this time, the front end of the inlet tube 244 is disposed below the rear end of the inlet tube 244, thereby applying the greatest restoring force to the front end of the inlet tube cover 245, and the front end of the inlet tube cover 245 is more firmly brought into closer contact with the inlet tube 244.

FIG. 17 illustrates an assembly method of the inner board 243 and the inlet tube cover 245. Referring to FIG. 17a, the inlet tube cover 245 is inserted into the inlet tube cover insertion groove 2462 of the inlet tube cover fixing member 246 disposed on the inner board 243. Referring to FIG. 17b, the inner board 243 to which the inlet tube cover 245 is coupled is in close contact with the upper surface of the dust collecting body 241. At this time, a support protrusion presses the connecting portion 2451 of the inlet tube cover, so that a stronger restoring force may be applied to the closing portion 2452.

The support protrusion 2422 is a component supporting the inlet tube cover 245. The support protrusion extends downward from the board and together with the inlet tube cover fixing member 146, the support protrusion is fitting and coupling the inlet tube cover 245.

The support protrusion extends downward from the inner board 143.

The lower surface of the inlet tube cover 245 is supported in close contact with the inner surface of the inlet tube cover fixing member 146. An upper surface of the inlet tube cover 245 is supported by the support protrusion.

The support protrusion is disposed on a radially outer side of the inlet tube 144. The support protrusion may extend along a circumferential direction of the inlet tube 144.

Referring to FIG. 18, according to another embodiment of the present invention, the cleaner station includes a magnetic member 2454 and a mating member 2444.

The inlet tube cover 245 includes the magnetic member 2454 for generating a magnetic field. The inlet tube 244 includes a mating member 2444 that generates attraction in response to a magnetic field.

The magnetic member 2454 is a component that generates a magnetic field, and may be a magnet.

The magnetic member 2454 may be disposed by inserting a separate object into the inlet tube cover 245. Alternatively, the magnetic member 2454 may be formed in a thin plate shape and disposed on an upper surface of the inlet tube cover 245. Alternatively, the inlet tube cover 245 itself may be formed of a magnetic material.

The magnetic member 2454 may be disposed on an outer periphery of the inlet tube cover 245. For example, the magnetic member 2454 may be disposed at an end farthest from the connecting portion of the inlet tube cover 245. Alternatively, the magnetic member 2454 may be disposed along an outer peripheral surface of the inlet tube cover 245.

The mating member 2444 is disposed at a lower end of the inlet tube. The mating member 2444 faces the magnetic member 2454.

The mating member 2444 may be a magnet having a polarity different from that of the magnetic member 2454. Alternatively, the mating member 2444 may be made of a magnetizable metal material such as an iron core.

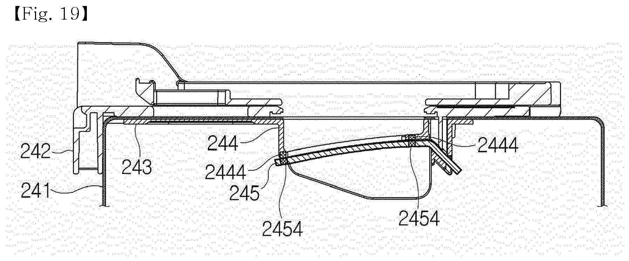

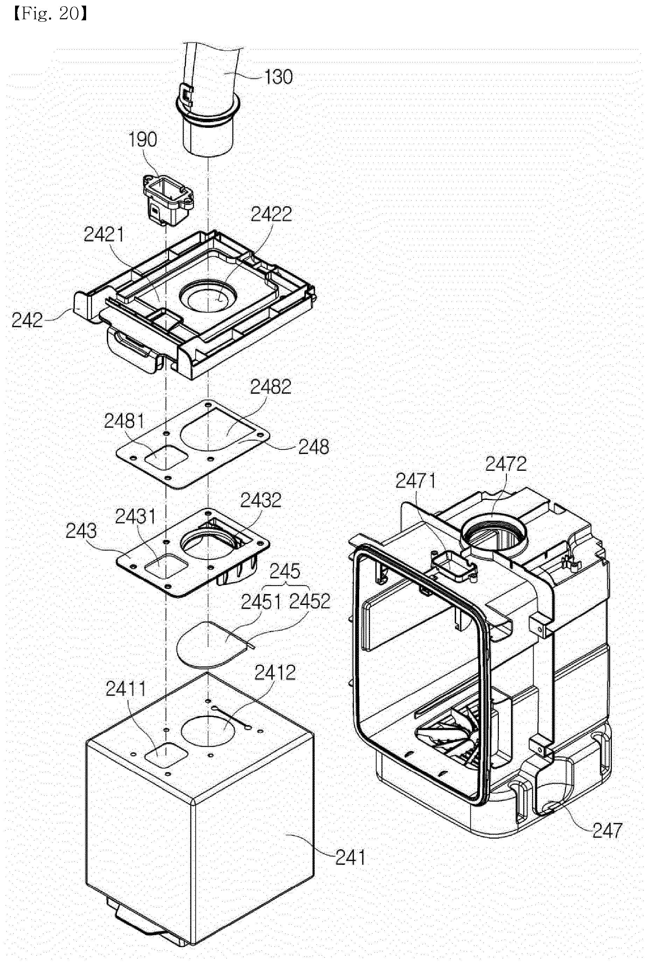

Referring to FIGS. 19 and 20, the cleaner station according to another embodiment of the present invention includes an adhesive member 248.

The adhesive member 248 is a component for more firmly fixing the dust collecting body 241 and the inner board 243 and sealing a gap between the dust collecting body 241 and the inner board 243.

The adhesive member 248 is disposed between the dust collecting body 241 and the inner board 243. Specifically, an upper surface of the adhesive member 248 is in close contact with an inner surface of the dust collecting body 241, and a lower surface thereof is in close contact with an upper surface of the inner board 243.

The adhesive member 248 may be formed in a shape corresponding to the inner board 243.

The adhesive member 248 includes a hole 2481 penetrated at a position vertically overlapping with the sterilizing light irradiating part 2411 of the dust collecting body and the sterilizing light irradiating part 2431 of the inner board. The sterilizing light passes through the hole 2481 and is irradiated into the dust collecting body 241.

The adhesive member 248 includes a hole 2482 penetrating at a position vertically overlapping with a dust inlet 2412 of the dust collecting body and a dust inlet 2432 of the inner board. The dust passes through the hole 2482 and is introduced into the dust collecting body 241.

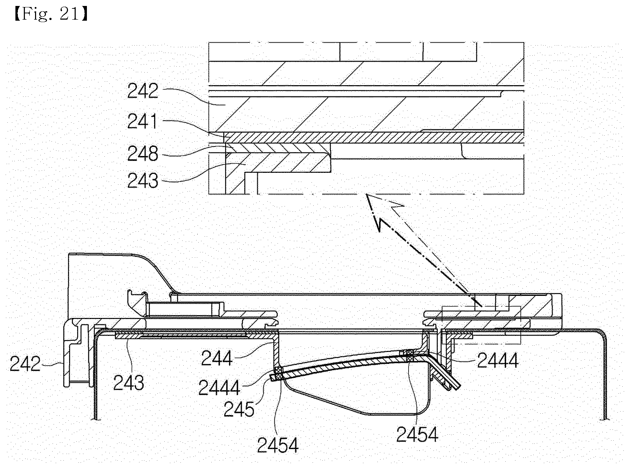

Referring to FIGS. 21 to 23, the cleaner station according to another embodiment of the present invention includes a gasket 249.

The gasket 249 is a component that seals a gap between the inlet tube 244 and the inlet tube cover 245.

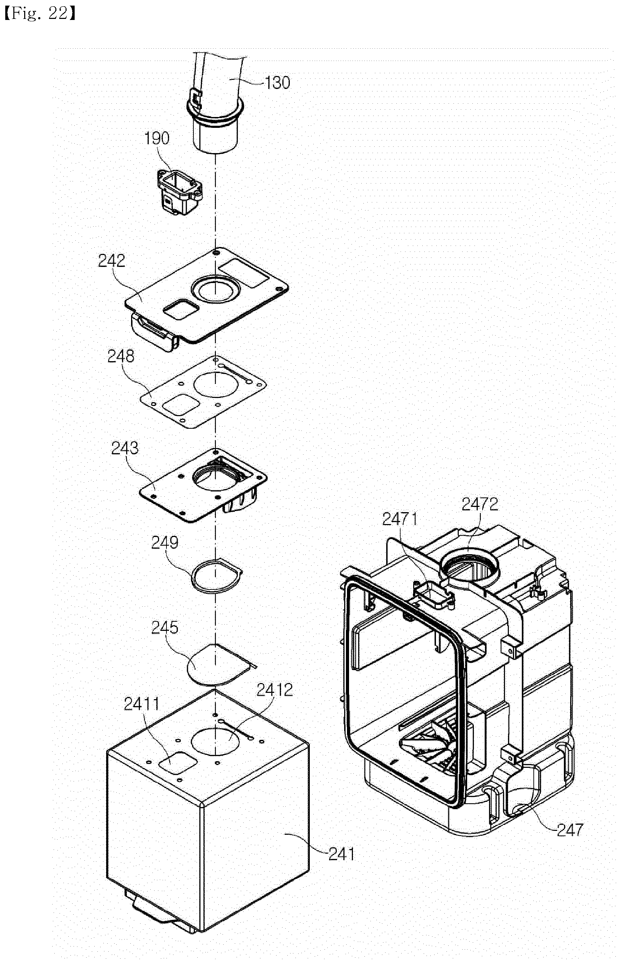

One side of the gasket 249 is in close contact with the inlet tube 244, and the other side thereof is in close contact with the inlet tube cover 245. Referring to FIG. 22, an upper surface of the gasket 249 is in close contact with the lower surface of the inlet tube 244, and a lower surface of the gasket 249 is in close contact with an upper surface of the inlet tube cover 245.

An adhesive is applied to the upper surface of the gasket 249, and the upper surface of the gasket 249 may be firmly fixed to the inlet tube 244 by the adhesive.

The gasket 249 extends along a circumferential direction of the inlet tube 244. The gasket 249 may be formed in a ring shape having a hollow.

The gasket 249 may be formed in a shape corresponding to a shape of an end of the inlet tube 244. Accordingly, the upper surface of the gasket 249 may be in surface contact with the lower surface of the inlet tube 244.

The gasket 249 may be formed of a soft rubber material. Alternatively, the gasket 249 may be formed of a porous foamed rubber or foamed Styrofoam material.

The inlet tube cover 245 and the gasket 249 may be formed of an elastic material. In this case, the strength of the gasket 249 may be weaker than the strength of the inlet tube cover 245.

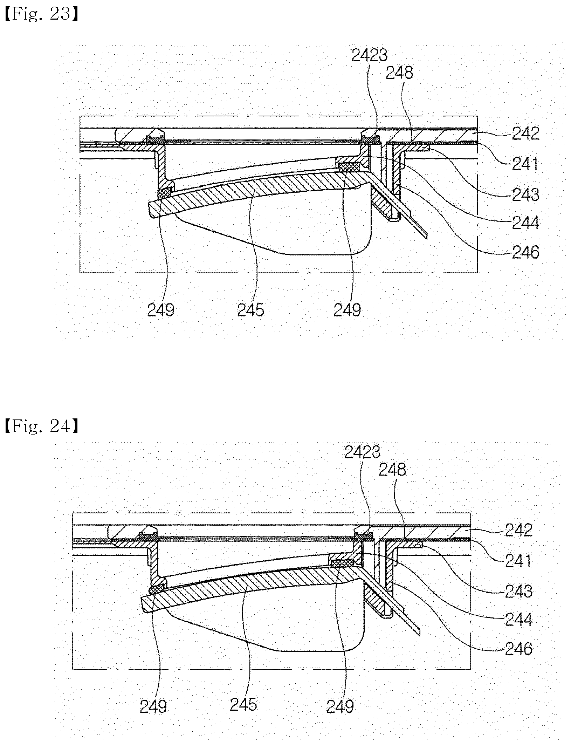

Referring to FIG. 23, the inlet tube cover 245 may apply pressure to the gasket 249 by a restoring force. At this time, since the strength of the gasket 249 is weaker than that of the inlet tube cover 245, the gasket 249 may be deformed whereas the inlet tube cover 245 is not deformed. Accordingly, as the gasket 249 is deformed, the gasket 249 is more closely attached to the inlet tube cover 245, thereby completely removing a gap between the gasket 249 and the inlet tube cover 245.

The inlet tube 244 may include a gasket insertion groove 2445. The gasket insertion groove 2445 is a component in which the gasket 249 can be seated.

The gasket insertion groove 2445 is formed by being depressed inwardly from an end adjacent to the inlet tube cover 245, and at least a portion of the gasket 249 is inserted therein.

Referring to FIG. 23, the gasket insertion groove 2445 may be formed at a lower end of the inlet tube 244. The gasket insertion groove 2445 may be formed by being depressed upwardly.

At least a portion of the gasket 249 may be inserted into the gasket insertion groove 2445.

The inlet tube 244 may include a gasket support protrusion 2446. The gasket support protrusion 2446 is a component that supports the gasket 249 and maintains a shape of the gasket 249.

The gasket support protrusion 2446 protrudes from one side of the inlet tube and is in close contact with an inner circumferential surface of the gasket 249.

Referring to FIG. 23, the gasket support protrusion 2446 may protrude downward from a lower surface of the inlet tube 244. The gasket support protrusion 2446 may extend along an inner circumferential surface of the gasket 249. The gasket support protrusion 2446 may be formed in a ring shape along the inner circumferential surface of the gasket 249.

Since the gasket support protrusion 2446 is in close contact with the inner circumferential surface of the gasket 249 to support the gasket 249, it is possible to prevent the gasket 249 having a weak strength from being easily deformed and having reduced sealing performance.

According to the embodiment of the present invention, the gasket 249 and the inlet tube 244 may be formed at the same time.

The gasket 249 may be coupled to the inlet tube 244. Specifically, together with the inlet tube 244, the gasket 249 may be formed by double injection. Alternatively, the gasket 249 may be formed by insert-injection molding into the inlet tube 244.

In this case, the gasket 249 is fixed to the inlet tube 244 and cannot be moved. When the inlet tube cover 245 rotates so that an upper surface of the inlet tube cover 245 and a lower surface of the gasket 249 come into close contact with each other, sealing is achieved.

According to the present embodiment, the gasket 249 is firmly coupled to the inlet tube 244, thereby improving durability.

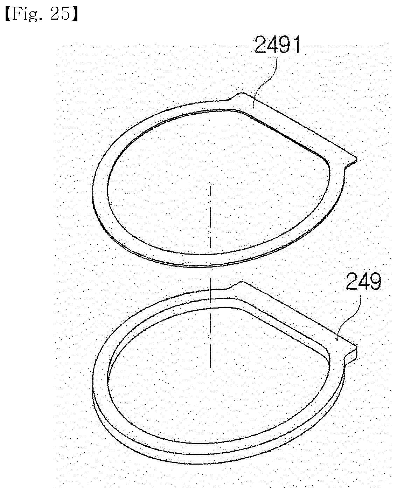

Referring to FIGS. 24 and 25, according to the embodiment of the present invention, the gasket may be fixed to the inlet tube by an adhesive member.

The gasket 249 may be coupled to the inlet tube 244. Specifically, the gasket 249 may be coupled to the inlet tube 244 by an adhesive member 2491.

Referring to FIG. 24, the adhesive member 2491 may be an adhesive tape. Alternatively, the adhesive member 2491 may be an adhesive.

In this case, the gasket 249 is fixed to the inlet tube 244 and cannot be moved. When the inlet tube cover 245 rotates so that an upper surface of the inlet tube cover 245 and a lower surface of the gasket 249 come into close contact with each other, sealing is achieved.

According to the present embodiment, when a life of the gasket 249 is expired or a part of the gasket 249 is separated from the inlet tube 244, there is an advantage that the gasket 249 may be easily replaced.

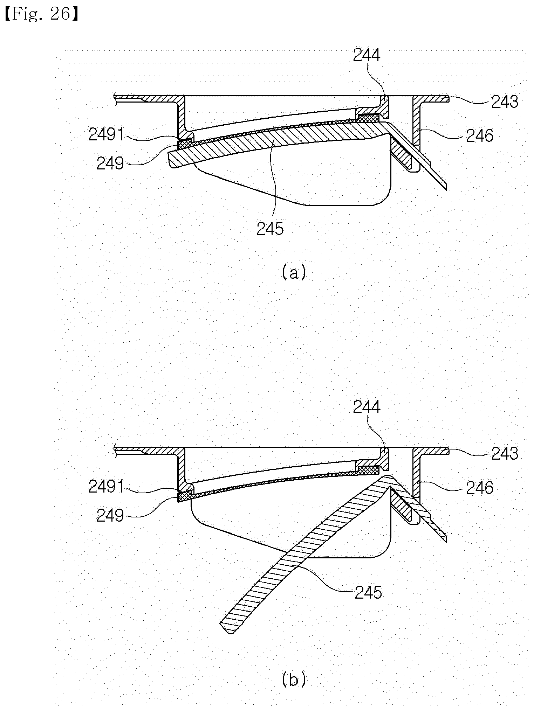

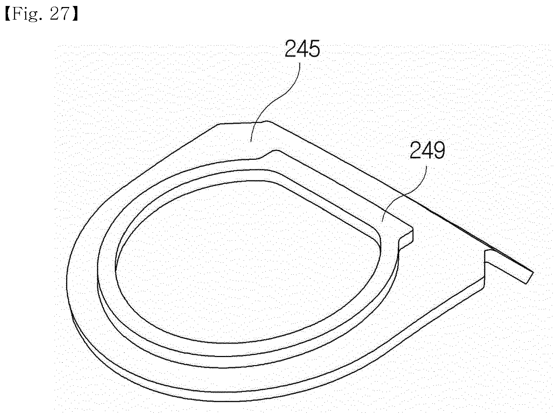

Referring to FIGS. 26 and 27, according to the embodiment of the present invention, the gasket 249 may be formed simultaneously with the inlet tube cover 245.

The gasket is coupled to the inlet tube cover 245 to move together with the inlet tube cover 245. Specifically, the gasket 249 may be formed by double injection together with the inlet tube cover 245. Alternatively, the gasket 249 may be formed by insert-injection molding into the inlet tube cover 245.

In this case, the gasket 249 may be fixed to the inlet tube cover 245 and move together with the inlet tube cover. When the inlet tube cover 245 rotates so that the upper surface of the gasket 249 is in close contact with the lower surface of the inlet tube 244, sealing is achieved.

According to the present embodiment, the gasket 249 is coupled to the inlet tube cover 245 and may be replaced together with the inlet tube cover 245, there is an advantage in that the gasket 249 and the inlet tube cover 245, which are consumables, may be replaced at once.

The operational effects of the cleaner station 100 according to the present disclosure configured as described above will be described.

A cleaner station 100 includes an inlet tube cover 245. The inlet tube cover 245 is disposed at an end of the inlet tube 244 and opens and closes the inlet tube 244. When a dust collecting motor is operated, the inlet tube cover 245 is deformed inward and opens the inlet tube 244, and when the dust collecting motor is stopped, the inlet tube cover 245 is restored by elasticity and closes the inlet tube 244. Therefore, by closing the inlet tube 244 when the dust collecting part 240 is separated from the cleaner station 100, it is possible to prevent dust from scattering, flying insects from escaping and an ordor from spreading.

An end of the inlet tube 244 has a specific curvature. The curvature may include a first curvature and a second curvature intersecting the first curvature. Accordingly, as the inlet tube cover 245 is double deformed by the first curvature and the second curvature, there are advantages in that sagging does not occur due to its own weight, and the inlet tube 244 is firmly sealed.

In addition, the dust collecting part 240 further includes a rib 1462 for pressing and deforming the inlet tube cover 245. Accordingly, the inlet tube cover 245 may be in close contact with an end of the inlet tube 244 while being deformed to form a first curvature and a second curvature.

In addition, the inlet tube cover 245 has an open front, and side walls 2461 are disposed on both sides of the inlet tube 244. The air introduced through the inlet tube 244 cannot flow backward by the inlet tube cover 245 and cannot flow laterally by the side wall 2461, so it must flow forward. A sterilizing light irradiating part is disposed in front of the inlet tube 244 to irradiate the sterilizing light, therefore there is also an advantage of sterilizing by killing microorganisms and winged insects.

In addition, although the preferred embodiments of this specification have been illustrated and described above, this specification is not limited to the above-described specific embodiments, and a person having ordinary skill in the art to which the present invention pertains may modify the present invention in various ways without departing from the gist of the present invention in the claims. Such modified embodiments should not be individually understood from the technical spirit or prospect of the present invention.

## Claims

1. A cleaner station comprising:
a suction flow path having an inlet end communicating with a dust bin of a cleaner; and
a dust collecting part connected to an outlet end of the suction flow path and collecting dust inside the dust bin of the cleaner,
wherein the dust collecting part comprises:
a dust collecting body having a dust inlet connected to the outlet end of the suction flow path;
an inlet tube extending inward from a circumferential surface of the dust inlet and having an end forming a curvature circle with a specific curvature when viewed from one side; and
an inlet tube cover disposed at the end of the inlet tube and opening and closing the inlet tube.

2. The cleaner station of claim 1,
wherein the inlet tube comprises:
a first curvature circle; and
a second curvature circle intersecting the first curvature circle.

3. The cleaner station of claim 1,
wherein the dust collecting part comprises:
a board disposed on an upper surface of the dust collecting body and having the dust inlet formed on one side thereof, and
wherein the inlet tube extends downward from the board.

4. The cleaner station of claim 3,
wherein the dust collecting part comprises:
an inlet tube cover fixing member fixing one side of the inlet tube cover to the board and bringing the inlet tube cover into closer contact with the end of the inlet tube.

5. The cleaner station of claim 4,
wherein the dust collecting part comprises:
a rib protruding from the inlet tube cover fixing member and having an end thereof supporting the inlet tube cover.

6. The cleaner station of claim 5,
wherein the rib presses and deforms the inlet tube cover.

7. The cleaner station of claim 6,
wherein the rib is disposed on a radially outer side of the suction flow path.

8. The cleaner station of claim 4,
wherein the inlet tube cover comprises:
a connecting portion coupled to the inlet tube;
a closing portion for opening and closing an end of the inlet tube; and
a bent portion disposed between the connecting portion and the closing portion.

9. The cleaner station of claim 8,
wherein the inlet tube cover fixing member comprises:
an inlet tube cover insertion groove into which at least a portion of the inlet tube cover is inserted.

10. The cleaner station of claim 8,
wherein the dust collecting part comprises:
a support protrusion extending downward from the board and, together with the inlet tube cover fixing member, fitting and coupling the the inlet tube cover to the dust collecting part.

11. The cleaner station of claim 1,
wherein the dust collecting part comprises:
a side wall disposed on one lateral side of the dust inlet and extending into an inner space thereof.

12. The cleaner station of claim 1,
wherein the dust collecting part comprises:
a suction flow path sealer extending radially inward from the dust inlet and having an inner end in close contact with the suction flow path.

13. The cleaner station of claim 1, further comprising:
a sterilizing light emitting member disposed on an outside of the dust collecting body,
wherein the dust collecting part comprises:
a sterilizing light irradiating part transmitting sterilizing light irradiated from the sterilizing light emitting member into the dust collecting body.

14. The cleaner station of claim 1,
wherein the inlet tube cover comprises:
a magnetic member generating a magnetic field, and
wherein the inlet tube comprises:
a mating member for generating attraction in response to the magnetic field.

15. The cleaner station of claim 1, comprising:
an adhesive member having an upper surface in close contact with an inner surface of the dust collecting body and a lower surface in close contact with an upper surface of an inner board.

16. The cleaner station of claim 1,
wherein the dust collecting part comprises:
a gasket having one side in close contact with the inlet tube and another side in close contact with the inlet tube cover.

17. The cleaner station of claim 16,
wherein the inlet tube comprises:
a gasket insertion groove formed by being recessed inward from an end adjacent to the inlet tube cover and into which at least a portion of the gasket is inserted.

18. The cleaner station of claim 16,
wherein the inlet tube comprises a gasket support protrusion that protrudes from one side and is in close contact with an inner circumferential surface of the gasket.

19. The cleaner station of claim 16,
wherein the inlet tube cover and the gasket are made of an elastic material, while a strength of the gasket is weaker than a strength of the inlet tube cover.

20. The cleaner station of claim 16,
wherein the gasket is coupled to the inlet tube.

21. The cleaner station of claim 16,
wherein the gasket is coupled to the inlet tube cover and moves together with the inlet tube cover.
